Europäisches Patentamt
European Patent Office
Office européen des brevets

(19)

(11) Publication number: 0 059 554
A2

(12) # EUROPEAN PATENT APPLICATION

(21) Application number: 82300798.4

(22) Date of filing: 17.02.82

(51) Int. Cl.³: C 07 D 487/04
A 61 K 31/40
//(C07D487/04, 209/00, 205/00)

(30) Priority: 27.02.81 GB 8106358
12.06.81 GB 8118107

(43) Date of publication of application:
08.09.82 Bulletin 82/36

(84) Designated Contracting States:
CH DE FR GB IT LI NL SE

(71) Applicant: BEECHAM GROUP PLC
Beecham House Great West Road
Brentford Middlesex(GB)

(72) Inventor: Coulton, Steven
6 Badgers Close
Horsham W. Sussex(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Beta-lactam antibiotics, a process for their preparation and their use in pharmaceutical compositions.**

(57) The present invention relates to carbapenem derivatives, and, in particular, to a novel class of substituted carbapenem derivatives wherein the C-6 substituent is a substituted triazolyl ethyl group.

The present invention provides a compound of the formula (I) or a pharmaceutically acceptable salt or *in vivo* hydrolysable ester thereof:-

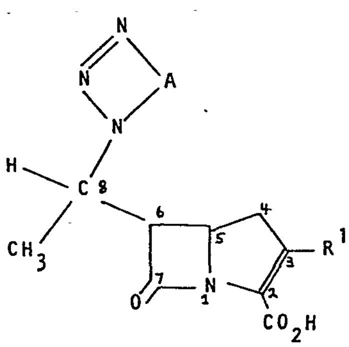

(1)

wherein $R^1$ is hydrogen, an organic radical or group $S(O)_nR^2$ wherein $R^2$ an organic radical and n is zero or one; and A is a divalent two atom radical forming the residue of a five membered ring wherein one of the atoms is optionally substituted carbon and the second atom is an optionally substituted carbon or nitrogen atom.

EP 0 059 554 A2

Croydon Printing Company Ltd.

## β-Lactam Antibiotics, a process for their preparation and their use in pharmaceutical compositions

The present invention relates to carbapenem derivatives, and, in particular, to a novel class of substituted carbapenem derivatives wherein the C-6 substituent is a substituted triazolyl ethyl group. This invention further relates to processes for their preparation and to compositions containing them. These derivatives are of use in the treatment of bacterial infection.

Accordingly, the present invention provides a compound of the formula (I) or a pharmaceutically acceptable salt or *in vivo* hydrolysable ester thereof:

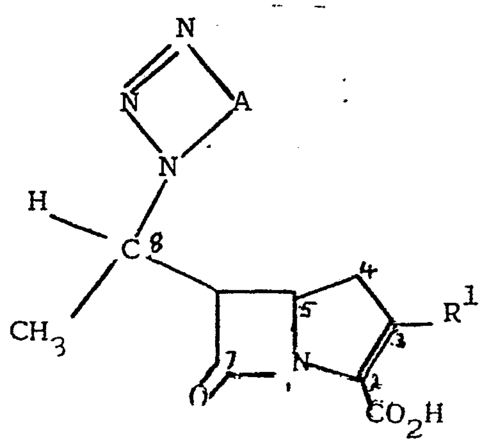

(I)

wherein $R^1$ is hydrogen, an organic radical or group $S(O)_nR^2$ wherein $R^2$ an organic radical and n is zero or one; and A is a divalent two atom radical forming the residue of a five membered ring wherein one of the atoms is optionally substituted carbon and the second atom is an optionally substituted carbon or nitrogen atom.

Suitably the divalent two atom radical completes an optionally mono-substituted triazole, tetrazole, dihydrotriazole, dihydrotetrazole, or a disubstituted triazole or dihydrotriazole ring.

More suitably the divalent two atom radical completes a mono or di-substituted triazole or dihydrotriazole ring.

Preferably the divalent two atom radical completes a mono- or di-substituted triazole ring.

Suitably the substituent will be a conjugating group, such as, for example, a carboxylic acid group or an ester or amide derivative thereof or a nitrile group.

Suitable compounds of formula (I) include those wherein $R^1$ is hydrogen, methyl, ethyl, n-or iso-propyl or n-, sec-, tert- or iso-butyl. Further suitable compounds of formula (I) are those wherein $R^1$ is a group of formula $SR^2$ wherein $R^2$ is $C_{1-6}$ alkyl, $C_{2-6}$ alkenyl, $C_{2-6}$ alkynyl, aryl, or a heterocyclyl, heterocyclyl $C_{1-6}$ alkyl, heteroaryl $C_{1-6}$ alkyl or heteroaryl group wherein the hetero atom or hetero atoms in the above named heteroaryl and/or heterocycyl moieties are selected from 1-4 oxygen, nitrogen or sulphur atoms; any of such $R^2$ groups being optionally substituted.

When $R^2$ is a $C_{1-6}$ alkyl group suitable substituents include amino, di-$(C_{1-6})$ alkylamino, $C_{1-6}$ alkylamino, acylamino, hydroxy, $C_{1-6}$ alkanoyloxy, $C_{1-6}$ alkoxy, benzoyl, $C_{1-6}$ alkanoyl or carboxy or an ester or salt thereof.

More suitably such alkyl groups contain up to 4 carbon atoms, for example $R^2$ aptly may be methyl, ethyl, propyl, butyl, aminoethyl, aminopropyl. aminobutyl, hydropropyl, hydroxypropyl, hydroxybutyl, methoxymethyl, methoxyethyl, ethoxymethyl, ethoxyethyl, propoxypropyl, methoxypropyl, methoxybutyl, acetoxymethyl, acetoxyethyl, propionoxymethyl, propionoxyethyl, phenacyl, acetylmethyl, acetylethyl, propionylmethyl, propionylethyl, carboxymethyl or pharmaceutically acceptable salt thereof, methoxycarbonylmethyl, ethoxycarbonylmethyl, methoxycarbonylethyl,

methoxycarbonylpropyl, methoxycarbonylbutyl, ethoxycarbonylethyl, carboxyethyl or pharmaceutically acceptable salt thereof, carboxypropyl or pharmaceutically acceptable salt thereof, carboxybutyl or pharmaceutically acceptable salt thereof, acetamidoethyl and propionamidoethyl. Of these values methyl, ethyl, propyl, butyl, 2-aminoethyl and acetamidoethyl are preferred.

When $R^2$ is a $C_{2-6}$ alkenyl group wherein the double bond is not present on the carbon adjacent to the sulphur atom, suitable substituents include carboxy or an ester or pharmaceutically acceptable salt thereof, or hydroxy or $C_{1-6}$ alkoxy.

When $R^2$ is a $C_{2-6}$ alkenyl group wherein the double bond is present on the carbon adjacent to the sulphur atom, suitable substituents include carboxy or an ester or pharmaceutically acceptable salt thereof.

More suitably $R^2$ is propenyl, butenyl and $CH=CH-CO_2H$ or a pharmaceutically acceptable salt or ester thereof, for example the sodium or potassium salt or the methyl, ethyl, propyl, aminoethyl, aminopropyl, benzyl or p-nitrobenzyl ester.

In another aspect preferred values for $R^2$ include the acetamidoethenyl and propionamidoethenyl groups.

Suitable aryl, heteroaryl and heterocyclyl groups for use in the substituent $R^2$ include phenyl, naphthyl, pyrrolyl, furyl, tetrazolyl, thienyl, indolyl, thionaphthyl, benzofuryl, imidazolyl, thiazolyl, or any of such groups substituted by one or more groups selected from $C_{1-3}$ alkyl, phenyl, nitro and amino; or a phenyl group optionally substituted by halogen atom or a $C_{1-3}$ alkoxy, nitro or acetamido group.

When $R^2$ is an aryl $C_{1-6}$ alkyl, heteroaryl $C_{1-6}$ alkyl or heterocyclyl $C_{1-6}$ aryl group, more suitably the alkyl

moiety is a methylene or ethylene divalent radical. Suitable examples of such aryl and heteroaryl moieties are phenyl optionally substituted by one or more substituents selected from a halogen atom or a $C_{1-3}$ alkoxy, nitro or acetamido group; pyrrolyl optionally substituted by a phenyl or $C_{1-3}$ alkyl group; thienyl optionally substituted by a phenyl or $C_{1-3}$ alkyl group; furyl optionally substituted by a phenyl or $C_{1-3}$ alkyl group; tetrazolyl optionally substituted by phenyl or a $C_{1-3}$ alkyl group; imidazolyl optionally substituted by one or more groups; selected from phenyl, nitro, amino, $C_{1-3}$ alkyl; and thiazolyl optionally substituted by one or more groups selected from phenyl, nitro, amino and $C_{1-3}$ alkyl.

More suitably $R^2$ is a benzyl, bromobenzyl, chlorobenzyl, flurobenzyl, methoxybenzyl, nitrobenzyl, acetamidobenzyl, thiazolylmethyl, aminothiazolymethyl, nitrothiazolymethyl or phenylthiazolylmethyl group.

Suitably also $R^2$ is a phenethyl, pyrrolylethyl or optionally substituted tetrazolylethyl or imidazolylethyl. In a suitable aspect the imidazolyl ring may be substituted at the C-2 position (that is the carbon atom α to the two nitrogen atoms) by a $C_{1-3}$ alkyl or phenyl group. In another aspect the imidazolyl ring may be further substituted at the C-4 position or the C-5 position by a $C_{1-3}$ alkyl, phenyl, nitro or amino groups; preferably such substituents are on the C-4 position and the C-5 position is unsubstituted; alternatively such substituents are on the C-5 position and the C-4 position is unsubstituted.

In a further aspect $R^2$ is a $C_{1-6}$ alkanoyl, aralkanoyl, aroxyalkanoyl or aroyl group, for example acetyl, phenylacetyl, phenoxyacetyl or benzoyl. Of these acetyl is preferred.

Suitably $R^2$ is phenyl optionally substituted by a halogen atom or a $C_{1-3}$ alkyl, $C_{1-3}$ alkoxy, nitro or acetamido group. Suitably $R^2$ is a 5- or 6-membered aromatic heterocyclic group containing 1 or 2 nitrogen atoms such as the pyridyl, pyrimidyl, pyrrolyl and imidazoyl ring systems, for example 2-pyridyl, 3-pyridyl, 4-pyridyl, 2-pyrimidinyl, 4-pyrimidinyl, 5-pyrimidinyl, 2-pyrrolyl, 3-pyrrolyl, 2-imidazolyl and 4-imidazolyl. Of these the pyridyl and pyrimidinyl ring systems are favoured, the pyrimidinyl ring being preferred and in particular the 2-pyrimidinyl and 4-pyrimidinyl ring systems. Suitably such aromatic heterocyclic ring systems are optionally substituted by one or more $C_{1-4}$ alkyl or $C_{1-4}$ alkanoyloxy groups.

Suitable salts of the compounds of formula (I) include salts e.g. aluminium, alkali metal salts such as sodium or potassium alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylamine, N,N-dibenzlethylenediamine, 1-ephenamine, N-ethylpiperidine, N-benzyl-β-phenethylamine, dehydroabietylamine, N,$N^1$-bisdehudroabietylamine, ethylenediamine, or bases of the pyridine type such as pyndine, collidine, or quinoline.

Suitable <u>in vivo</u> hydrolyzable esters of the compound of formula (I) include acyloxyalkyl groups such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl,

dimethylaminoethyl, or diethylaminoethyl; and lactone groups such as phthalidyl or dimethoxyphthalidyl.

It is realised that in the compounds of this invention the hydrogen atoms at the C-5 and C-6 carbon atoms may be in the cis or trans configuration; preferably the hydrogen atoms at the C-5 and C-6 carbon atoms will be in the trans configuration with the configuration 5R, 6S. When in the cis configuration the preferred stereochemistry is 5R,6R.

It is further realised that the carbon atom assigned as C-8 is also a centre of optical activity and may exist in either the R or S stereochemical configuration. Most suitably the C-8 carbon atom will be in the R configuration.

Those $R^2$ substituents containing a double bond may be present in the E or Z configuration, for example, $R^2$ may suitably be (E)-acetamidoethenyl and (Z)-acetamidoethenyl.

One preferred sub-group of compounds within the scope of the present invention is the compound of formula (II) or a pharmaceutically acceptable salt or *in vivo* hydrolysable ester thereof:

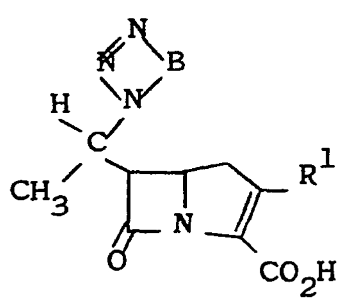

(II)

wherein $R^1$ is as defined hereinbefore with respect to formula (I) and B is a divalent radical of structure:

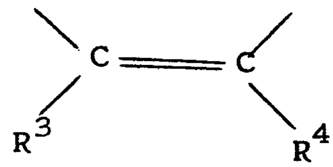

wherein $R^3$ represents a carboxylic acid group or an ester or amide derivative thereof or a nitrile group; and $R^4$ represents hydrogen, an optionally substituted hydrocarbon or heterocyclic group, a carboxylic acid group or an ester or amide derivative thereof or a nitrile group.

The radicals $R^3$ and $R^4$ may be the same or different and each is suitably a carboxylic acid or an ester group $-CO_2R^5$ wherein $R^5$ is an alkyl, cycloalkyl, alkenyl, alkynyl, aralkyl, aryl, heterocyclic or heterocyclyl alkyl group, any of which may be substituted. Suitable such $R^5$ groups include the $C_{1-6}$ alkyl group such as methyl, ethyl, n- or iso-propyl, n-, sec-, tert-, or iso- butyl, or substituted $C_{1-6}$ alkyl wherein the substituent is an amino group or a protected amino group, such as for example p-nitrobenzyloxycarbonylamino.

Preferably $R^3$ is a carboxylic acid group, or a salt, or methyl, ethyl, p-nitrobenzyl, 2-amino-ethyl or 2-(p-nitrobenzyloxycarbonylamino)ethyl ester thereof. Preferably $R^4$ is hydrogen.

Particular compounds within the scope of the present invention include:

sodium (5R,6S)-3-(2-acetamidoethylthio)-6-[(R)-1-(4,5-dimethoxycarbonyltriazol-1-yl)ethyl]-7-oxo-1-azabicyclo[3.2.0.]hept-2-ene-carboxylate;

disodium (5R,6S)-3-(2-acetamidoethylthio)-6-[(R)-1-(4-carboxytriazol-1-yl)ethyl]-7-oxo-1-azabicyclo[3.2.0] hept-2-ene-2-carboxylate.

sodium (5R,6S)-3-(E-2-acetamidoethenylthio)-6-[(R)-1-(4,5-dimethoxycarbonyltriazol-1-yl)ethyl]-7-oxo-1-azabicyclo [3.2.0.] hept-2-ene-2-carboxylate.

disodium (5R,6S)-3-[(E)-2-acetamidoethenylthio]-6-[(R)-1-(4-carboxytriazol-1-yl)ethyl]-7-oxo-1-azabicyclo [3.2.0.] hept-2-ene-2-carboxylate.

(5R,6S)-3-[(E)-2-acetamidoethenylthio]-6-[(R)-1-[4-(2-aminoethyloxycarbonyl)triazol-1-yl] ethyl]-7-oxo-1-azabicyclo [3.2.0.] hept-2-ene-2-carboxylic acid.

The compounds of the formula (I) may be employed in the treatment of bacterial infections, such as those due to Staphylococcus aureus, Escherichia coli and Klebsiella aerogenes. Thus the present invention provides a pharmaceutical composition which comprises a compound of the formula (I) and a pharmaceutically acceptable carrier.

The compositions may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters

such as glycerine, propylene glycol, or ethyl alcohol; preservatives, for example methyl or propyl p-hydroxybenzoate or sorbic acid, and if desired conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vaccuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Unit dose forms according to this invention will normally contain from 50 to 500 mgs of a compound of this invention, for example about 62.5, 100, 125, 150, 200, 250

or 300 mgs. Such compositions may be administered from 1 to 6 times a day or more conveniently 2, 3 or 4 times a day so that the total daily dose for a 70kg adult is about 200 to 2000 mgs, for example about 400, 600, 750, 1000 or 1500 mgs.

The compositions of this invention may be used to treat infections of the respiratory tract, urinary tract or soft tissues in humans, or mastitis in cattle.

The present invention also provides synergistic pharmaceutical compositions which comprise a pharmaceutical composition as hereinbefore described which also contains a penicillin or a cephalosporin.

Suitable penicillins for inclusion in the compositions of this invention include benzyl penicillin, phenoxymethyl-penicillin, ampicillin, or a pro-drug therefor, amoxycillin, or a pro-drug therefor, carbenicillin or a pro-drug therefor, ticarcillin or a pro-drug therefor, suncillin, sulbenicillin, azlocillin, mezlocillin, piperacillin or apalcillin.

Particularly suitable penicillins for inclusion in orally administrable compositions of this invention include ampicillin and its orally administrable pro-drugs, amoxycillin and its orally administrable pro-drugs and orally administrable pro-drugs or carbenicillin. Thus particularly suitable penicillins include ampicillin anhydrate, ampicillin trihydrate, sodium ampicillin, talampicillin hydrochloride, pivampicillin hydrochloride and bacampicillin hydrochloride; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion

in the orally administrable compositions of this invention is ampicillin trihydrate.

Particularly suitable penicillins for inclusion in injectably administrable compositions of this invention include injectable salts, such as the sodium salt of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusion in the injectably administrable compositions of this invention include cephaloridine, cefazolin and cephradine, generally as their pharmaceutically acceptable salt.

The weight ratio between compound of this invention and penicillin or cephalosporin is generally between from 10:1 to 1:10, more usually from 5:1 to 1:5 and normally from 3:1 to 1:3.

The penicillin or cephalosporin is generally utilised in its conventionally administered amount.

The invention also provides a method of treatment of bacterial infections in animals including man which comprises the administration of an antibacterially effective amount of a compound of the invention.

The present invention also provides a process for the preparation of a compound of the formula (I) or a pharmaceutically acceptable salt or in vivo hydrolysable ester thereof which process comprises reacting a compound of formula (III):

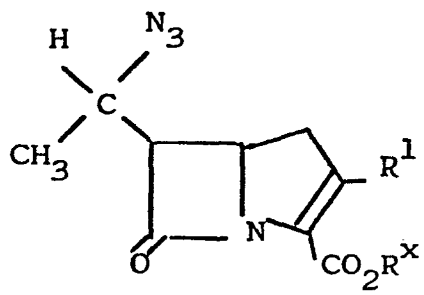

(III)

wherein $R^1$ is as defined with respect to formula (I) and wherein any reactive groups may be protected and $R^x$ is a carboxy protecting group.

with a dipolarophile; and thereafter where necessary carrying out one or more of the following steps:

i) removing any protecting groups on $R^1$, or the dipolarophile,

ii) removing the carboxy protecting group $R^x$,

iii) converting a free acid to an in vivo hydrolysable ester or a phrmaceutically acceptable salt.

Suitably the reaction is performed at an elevated temperature ie in the range 50-150°C. Conveniently the reaction is performed at the reflux temperature of the solvent employed in the reaction. Suitable solvents include aromatic hydrocarbons such as, for example, benzene and toluene, paraffins, and ethers such as tetrahydrofuran. The course of the reaction may be followed by conventional methods such as thin layer chromatography and terminated when an optimum quantity of product is present in the reaction mixture.

When used herein the term dipolarophile indicates an optionally substituted alkene, alkyne, nitrile, imine, and wherein any reactive substituents may be protected.

Suitably at least one substituent on the dipolarophile will be a conjugating group.

Preferably the dipolarophile will be a compound of formula IV:

$$R^3-C\equiv C-R^4 \qquad (IV)$$

wherein $R^3$ and $R^4$ are as defined with respect to formula (II) and wherein any reactive groups may be protected.

Suitably carboxyl-blocking derivatives for the group $-CO_2R^x$ in formula (III) include ester derivatives of the carboxylic acid. The derivative is preferably one which may readily be cleaved at a later stage of the reaction.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, o-nitrobenzyl, 4-pyridylmethyl, 2,2,2-trichloroethyl, 2,2,2-tribromoethyl, acetonyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, or an <u>in vivo</u> hydrolysable ester radical such as defined above.

A preferred group $R^x$ in the p-nitrobenzyl group.

The carboxyl group may be regenerated from any of the above esters by usual methods appropriate to the particular $R^x$ group, for example, acid - and base - catalysed hydrolysis, or by enzymically - catalysed hydrolysis, or by hydrogenolysis.

The starting material of formula (III) having sub-formula (V):

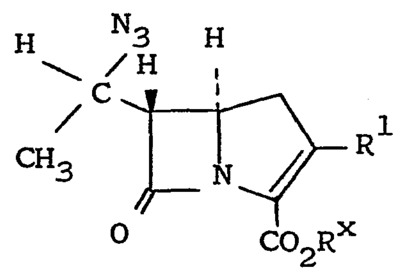

(V)

wherein $R^1$ is a defined with respect of formula (I) and wherein any reactive groups may be protected and $R^x$ is hydrogen or a carboxy blocking group are disclosed in U.K. Patent Application No. 8034453.

The starting material of formula (III) having sub-formula (VI):

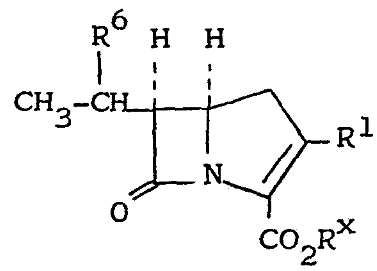

(VI)

wherein $R^1$ is as defined with respect to formula (I) and wherein any reactive groups may be protected, $R^x$ is hydrogen or a carboxy blocking group, and $R^6$ is an azido group may be prepared by a process which comprises the reaction of a compound of the formula (VII):

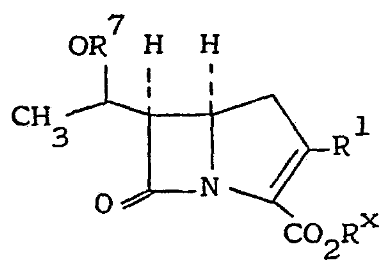

(VII)

wherein $R^1$ is as hereinbefore defined, $OR^4$ is a displaceable group, and $R^x$ is a hydrogen atom or a carboxy-blocking group; with an azide, and thereafter if necessary removing any carboxy blocking group.

Suitably the azide is present in the form of an inorganic azide such as sodium azide or lithium azide, or as an organic azide such as tetramethylguanidium azide. Care must be exercised when selecting an appropriate azide as many azides, particularly heavy metal azides are explosive.

Suitably $OR^7$ is a readily displaceable group such as a sulphonate ester for example p-nitrobenzenesulphonate or trifluoromethylsulphonate.

The reaction is performed in an inert organic solvent which is preferably aprotic and unreactive towards the reagents involved. Suitable solvents include dimethylformamide.

In addition to utility as an intermediate the compound of formula (VII) wherein $R^x$ represents hydrogen are of use in the treatment of bacterial infection. Reduction of the azido group $R^6$ leads to compounds also of use in the treatment of bacterial infection.

Accordingly the present invention also provides a compound of formula (VIII) or a pharmaceutically acceptable salt or _in vivo_ hydrolysable ester thereof:

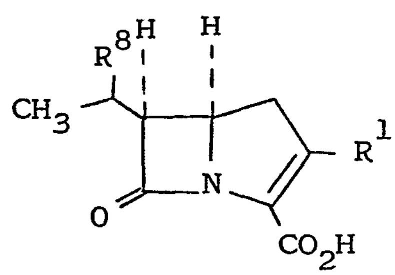

(VIII)

wherein $R^1$ is as defined with respect to formula (I) and $R^8$ is an azido or amino group.

Suitably $R^8$ is azido. Preferably $R^8$ is amino.

A compound of the formula (VIII) wherein $R^8$ is an azido group may be converted to a compound of the formula (VIII) wherein $R^8$ is an amino group by standard reduction methods, such as catalytic hydrogenation, for example using a transition metal catalyst such as palladium suitably palladium on carbon.

The present invention further provides a pharmaceutical composition which comprises a compound of formula (VIII) and a pharmaceutically acceptable carrier.

The following Examples illustrate the present invention.

Example 1

p-Nitrobenzyl (5R,6R)-3-(E-2-acetamidoethenylthio)-6-[(S)-1-p-nitrobenzenesulphonyloxyethyl]-7-oxo-1-azabicyclo[3.2.0]hept-2-ene-2-carboxylate

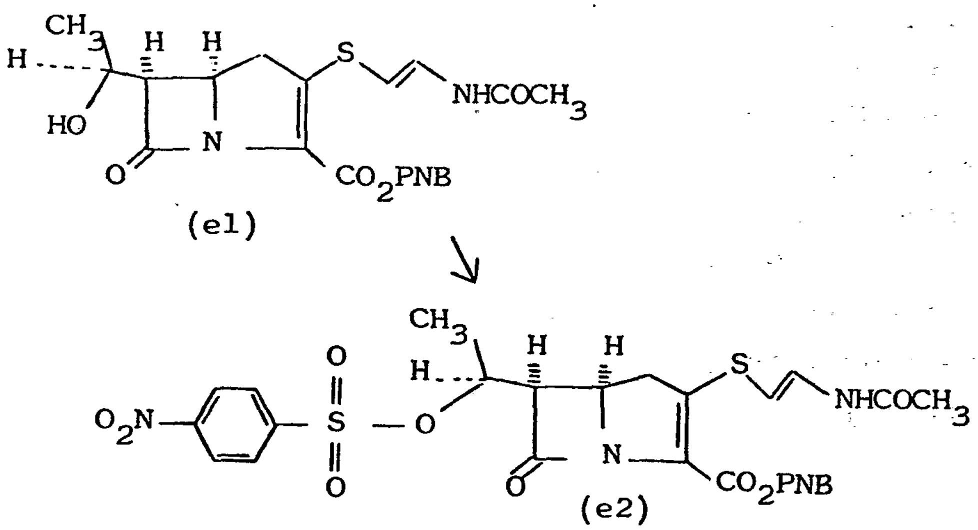

(e1)

(e2)

p-Nitrobenzyl (5R,6R)-3-(E-2-acetamidoethenylthio)-6-[(S)-1-hydroxyethyl]-7-oxo-1-azabicyclo[3.2.0]hept-2-ene-2-carboxylate (e1) (0.500 gm) was dissolved in dry tetrahydrofuran (125 ml) and cooled to $0^{o}C$. N-Methylmorpholine (0.452 gm), 4-dimethylaminopyridine (0.100 gm) and p-nitrobenzenesulphonyl chloride (1.00 gm) were added to the solution which was then stirred at room temperature for 18 hours. The solvent was removed at reduced pressure and the residue partitioned between ethyl acetate and water. The organic layer was washed with saturated sodium chloride solution and dried over anhydrous magnesium sulphate. Removal of the solvent at reduced pressure gave a pale yellow oil, which was chromatographed over silica gel (25 gm). Elution with ethyl acetate gave

the title compound (e2) as a pale yellow oil (89 mg). Crystallisation from chloroform afforded a white solid (56 mg), $\lambda_{max}$ (EtOH) 321 nm ($\varepsilon_m$ 13887), 254 nm ($\varepsilon_m$ 24698), $\nu_{max}$ ($CHCl_3$) 3420, 1775, 1699, 1620, 1608, 1522, 1351 $cm^{-1}$, $\delta_H$ ($CDCl_3$) 1.48 (3H, d, C$\underline{H}_3$CH), 2.13 (3H, s, COC$\underline{H}_3$), 3.0-3.4 (2H, $\underline{AB}$X, 4-C$\underline{H}_2$), 3.81 (1H, dd, $\underline{J}_{68}$ 9.2 Hz, $\underline{J}_{56}$ 5.8 Hz, 6-C$\underline{H}$), 4.2-4.4 (1H, dt, 5-C$\underline{H}$), 5.1-5.25 (1H, m, 8-C$\underline{H}$), 5.36 (2H, AB, C$\underline{H}_2$Ar), 5.89 (1H, d, S-C$\underline{H}$=C), 7.31 (1H, dd, C=C$\underline{H}$-NH), 7.59 (d, aromatic protons), 8.08 (d, aromatic protons), 8.20 (d, aromatic protons), 8.41 (d, aromatic protons), m/e (relative intensity %) 429.0965 ($C_{20}H_{19}N_3O_6S$ requires 429.0982), 203(20), 60(100), 57(70). Further elution with 25% ethanol/ethyl acetate gave unreacted starting material as a white solid from ethyl acetate (300 mg).

Example 2

p-Nitrobenzyl (5R,6R)-3-(E-2-acetamidoethenylthio)-6-[(R)-1-azidoethyl]-7-oxo-1-azabicyclo[3.2.0]hept-2-ene-2-carboxylate

(e2)

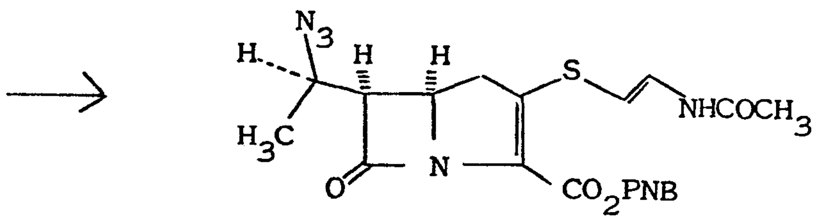

(e3)

The p-nitrobenzenesulphonate (e2) (56 mg) was dissolved in dry dimethylformamide (2 ml) and stirred with sodium azide (6.9 mg) for 2 hours at room temperature. The solvent was removed at reduced pressure and the residue partitioned between ethyl acetate and water. The organic layer was washed with saturated sodium chloride solution, dried over anhydrous magnesium sulphate and the solvent removed at reduced pressure to yield a yellow oil. This oil was chromatographed over silica gel (10 gm). Elution with a gradient of 0-2% ethanol/chloroform gave the title compound (e3) as a white solid, which was digested in ethyl acetate/ether and collected by filtration (7 mg), $\nu_{max}$ ($CHCl_3$) 3420, 2125, 1775, 1700, 1620, 1521, 1350, 1330 $cm^{-1}$, $\lambda_{max}$ (EtOH) 322 nm ($\varepsilon_m$ 16212), 262 nm ($\varepsilon_m$ 17128), $\delta_H$ ($CDCl_3$) 1.43 (3H, d, $C\underline{H}_3CH$), 2.09 (3H, s, $COC\underline{H}_3$), 2.9-3.35 (2H, $\underline{AB}X$, 4-$C\underline{H}_2$), 3.61 (1H, t, $\underline{J}_{68}$ 6.8 Hz, 6-$C\underline{H}$), 3.87 (1H, apparent quintet, 8-$C\underline{H}$),

4.25 (1H, dt, $\underline{J}_{56}$ 6.0 Hz, 5-C$\underline{H}$), 5.38 (2H, AB, C$\underline{H}_2$Ar), 5.94 (1H, d, S-C$\underline{H}$=C), 7.25 (1H, dd, C=C$\underline{H}$-N), 7.65 (3H, broad res. + d, N$\underline{H}$ + aromatic protons), 8.22 (2H, d, aromatic protons), m/e (relative intensity %) 472(1), 429(5), 361(5), 249(5), 207(5), 183(8), 136(15), 106(15), 84(100).

EXAMPLE 3

(a) p - Nitrobenzyl (5R,6S) - 3 - (2-acetamidoethylthio) - 6 - [(R) - 1 - (4,5 - dimethoxycarbonyltriazol - 1 - yl) ethyl] - 7 - oxo - 1 - azabicyclo [3.2.0.] hept - 2 - ene - 2 - carboxylate

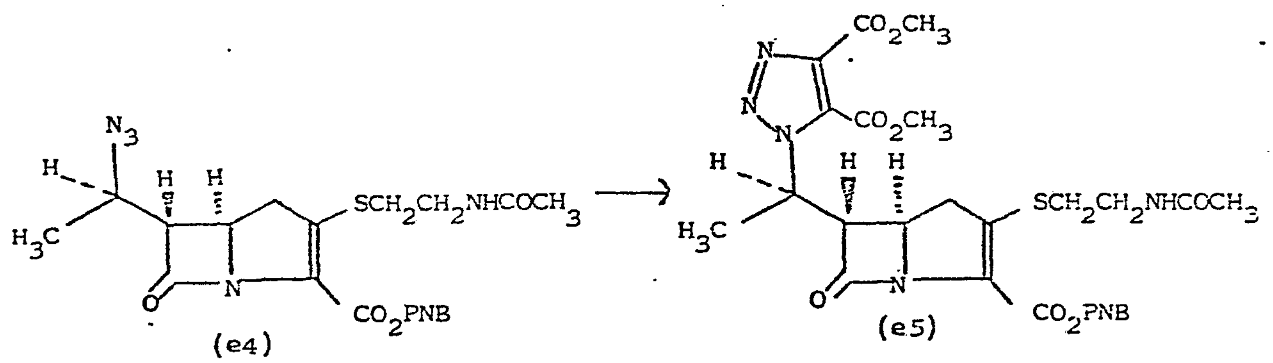

(e4) (e5)

p-Nitrobenzyl (5R,6S)-3-(2-acetamidoethylthio)-6-[(R)-1-azidoethyl]-7-oxo-1-azabicyclo[3.2.0.]hept-2-ene-2-carboxylate (55mg; 0.116mM) was suspended in dry toluene (15ml) and heated to reflux for 6 hours with dimethyl acetylenedicarboxylate (33mg; 0.232mM). The solution was then cooled to room temperature and the precipitated solid collected by filtration, washing with toluene, ethyl acetate and ether. The title compound was obtained as a pale yellow needle-shaped crystalline solid (51mg) λmax ($CH_3CN$) 318nm (εm13200), 267nm (εm11626), νmax (KBr) 3400, 1780, 1730, 1705, 1655, 1608, 1548, 1522, 1350, 1335$cm^{-1}$, $d_H$($d_7$-DMF) 8.28(2H,d, aromatic protons), 7.81 (2H,d, aromatic protons), 5.2-5.7 (3H,m,$C\underline{H}_2$Ar+8-C$\underline{H}$), 4.02 (3H,S,$CO_2C\underline{H}_3$), 3.93(3H,S,$CO_2C\underline{H}_3$), 1.87(3H,S,$COC\underline{H}_3$), 1.76 (3H,d, $\underline{J}$ 6.5$H_Z$, $C\underline{H}_3$CH), m/e (relative intensity %) 431(3), 363(20), 304(75), 154(80), 124(50), 86(100).

(b) Sodium (5R,6S) - 3 - (2-acetamidoethylthio) - 6 - [(R) - 1 - (4,5 - dimethoxycarbonyltriazol - 1 - yl) ethyl] - 7 - oxo - 1 - azabicyclo [3.2.o.] hept - 2 - ene - 2 - carboxylate.

(e5) ⟶ 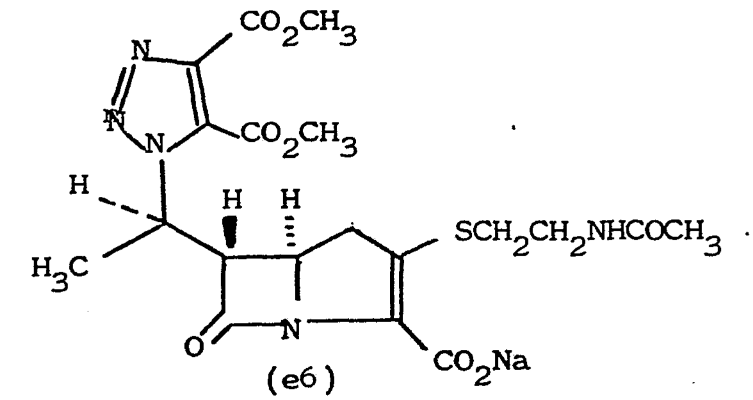

(e6)

The ester (e2)(15mg) was suspended in 30% aqueous 1,4-dioxan (15ml) and shaken for 3.5 hours with hydrogen in the presence of 5% palladium on carbon catalyst (23mg), at ambient temperature and pressure. Sodium bicarbonate (2.1mg) was added and the suspension filtered over celite, washing well with water (25ml). The filtrate was concentrated to approximately 20ml at reduced pressure and washed with ethyl acetate (3 x 50ml). The resulting aqueous solution was estimated to contain 7.3mg of the title compound (e6) based on $\epsilon$m8,000 at $\lambda$max 295nm in the ultra violet spectrum.

0059554

EXAMPLE 4

(a) p - Nitrobenzyl (5R,6S) - 3 - (2 - acetamidoethylthio) - 6 - [(R) - 1 - (4 - p - nitrobenzyloxycarbonyltriazol - 1 - yl) ethyl] - 7 - oxo - 1 - azabicyclo [3.2.0.] hept - 2 - ene - 2 - carboxylate and p - nitrobenzyl (5R,6S) - 3 - (2 - acetamidoethylthio) - 6 - [(R) - 1 - (5 - p - nitrobenzyloxycarbonyl-triazol - 1 - yl) ethyl] - 7 - oxo - 1 - azabicyclo [3.2.0.] hept - 2 - ene - 2 - carboxylate.

(e4) 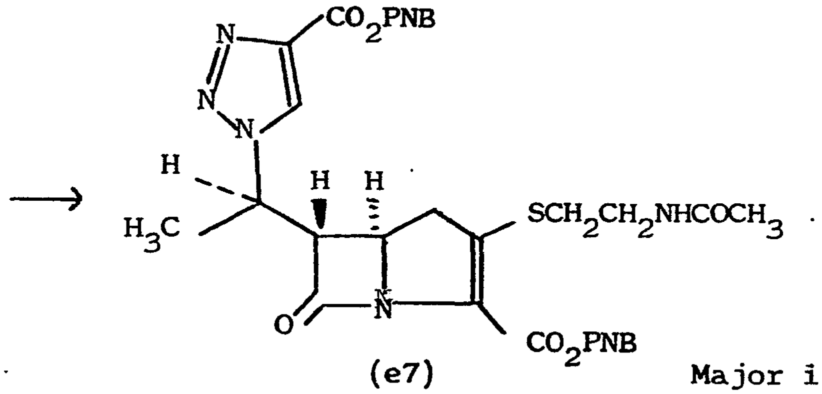

(e7) Major isomer

+

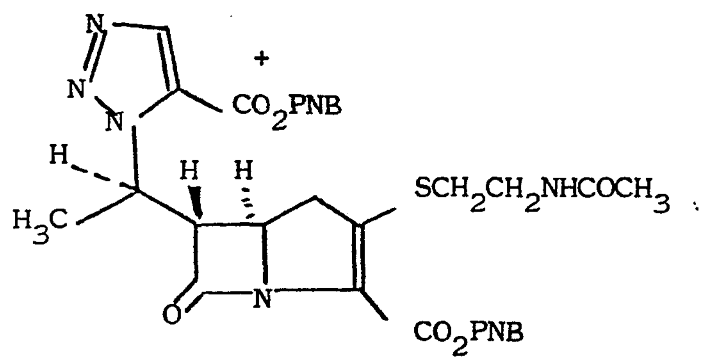

(e8) Minor isomer

The azido derivative (e4)(60mg;0.127mM) was suspended in dry tol`ene (15ml) and refluxed for 3 hours with p-nitrobenzyl propiolate (52mg,0.253mM), during which time the cycloaddition products crystallised out of solution. The white solid (48mg) was collected by filtration, λmax ($CH_3CN$) 316nm (€m13320),

267nm ( m20406), νmax (KBr)3400, 1778, 1738, 1700, 1660, 1608, 1520, 1350cm$^{-1}$, $d_H$ ($d_6$-acetone) 8.82(1H's, triazolyl CH), 8.25 (2xd, 4H, aromatic protons), 7.79(4H,d, aromatic protons), 7.38(1H, broad resonance, NH), 5.28-5.63(5H, AB+s+m, 2x$CH_2$Ar+8-CH), 4.27(1H, dt,5-CH), 4.10(1H, dd, $J_{56}$2.9$H_z$, $J_{68}$8.8$H_z$, 6-CH), 3.28-3.52(m,$NCH_2$+4-$CH_2$), 2.9-3.1(2H,m, $SCH_2$), 1.86 (3H,S, $COCH_3$), 1.82(3H,d,$CH_3$CH). Additional signals at 8.36(S, triazolyl CH) and 1.77(d, $CH_3$CH) in the N.M.R. spectrum indicated the presence of the minor isomer (e8)(15%).

(b) Disodium (5R,6S) - 3 - (2 - acetamidoethylthio) - 6 - [(R) - 1 - (4 - carboxytriazol - 1 - yl) ethyl] - 7 - oxo - 1 - azabicyclo [3.2.0.]hept - 2 - ene - 2 - carboxylate.

(e7) 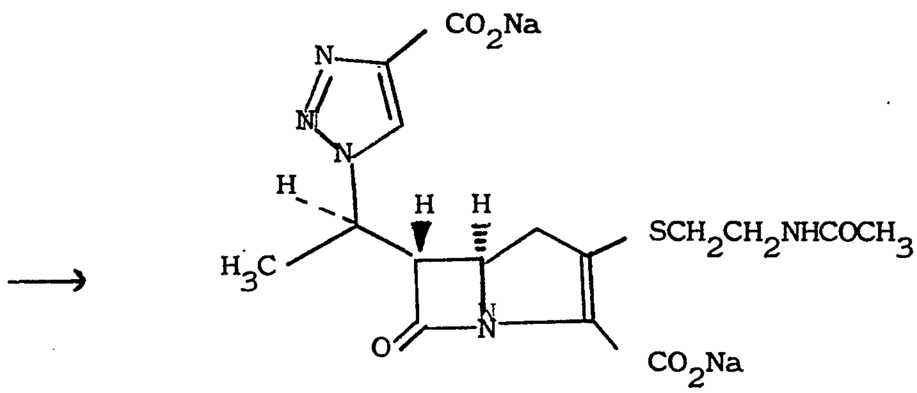

(e9)

The diester (e7)(12mg) was suspended in 30% aqueous 1,4-dioxan (15ml) and shaken for 3.5 hours with hydrogen in the presence of 5% palladium on carbon catalyst (18mg) at ambient temperature and pressure. Sodium bicarbonate (3mg) was added and the suspension filtered over Celite, washing well with water (25ml). The filtrate was concentrated to approximately 20ml at reduced pressure and washed with ethyl acetate (3 x 50ml). The resulting aqueous solution was estimated to contain 6.0mg of the title compound (e9) based on $\epsilon_m$8,000 at $\lambda_{max}$ 299nm in the ultra violet spectrum.

Example 5

a) p-Nitrobenzyl (5R,6S)-3-(E-2-acetamidoethenylthio)-6-[(R)-1-(4,5-dimethoxycarbonyltriazol-1-yl)ethyl]-7-oxo-1-azabicyclo[3.2.0.]hept-2-ene-2-carboxylate

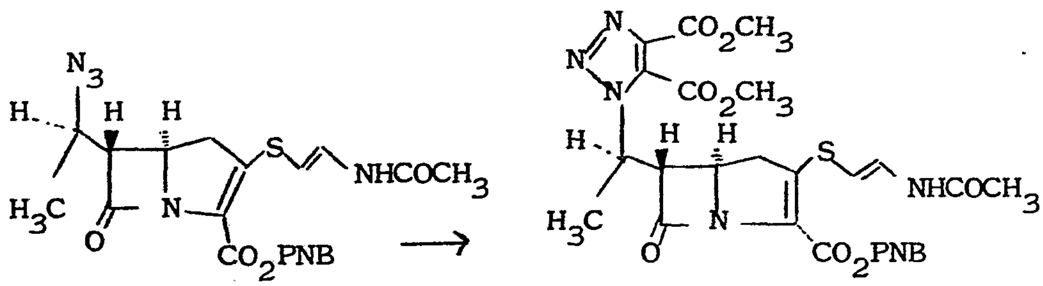

(e10) (e11)

p-Nitrobenzyl (5R,6S)-3-(E-2-acetamidoethenylthio)-6-[(R)-1-azidoethyl]-7-oxo-1-azabicyclo [3.2.0.]hept-2-ene-2-carboxylate (60 mg; 0.127 mM) was suspended in dry toluene (15 ml) and refluxed for 3 hours with dimethylacetylenedicarboxylate (36 mg; 0.254 mM). The solution was cooled to room temperature and the resulting precipitate collected by filtration. This solid was purified by column chromatography over silica gel (2 gm). Elution with ethyl acetate afforded the title compound as a white solid, which was digested in dry diethyl ether and collected by filtration (15 mg), $\nu_{max}$ (KBr) 3400, 1780, 1730, 1700, 1620 $cm^{-1}$, $\lambda_{max}$ ($CH_3CN$) 323 nm (Em 16,610), 262 nm (Em 18,090), 217 nm (24,775), $\delta_H$ ($d_7$-DMF) 10.48 (1H, d, N$\underline{H}$), 8.31 (2H, d, aromatic protons), 7.84 (2H, d, aromatic protons), 7.21 (1H, q, C=C$\underline{H}$NH), 5.98 (1H, d, C=C$\underline{H}$-S); 5.3-5.7 (3H, ABq +m, C$\underline{H}_2$Ar + 8-C$\underline{H}$), 4.38 (1H, dd, $\underline{J}$ 2.9Hz and 10.0 Hz 6-C$\underline{H}$), 4.21 (1H, dt, 5-C$\underline{H}$), 4.06 (3H, S, $CO_2C\underline{H}_3$); 3.97 (3H, S, $CO_2C\underline{H}_3$), 3.27 (2H, $\underline{AB}$X, 4-C$\underline{H}_2$), 2.03 (3H, S, COC$\underline{H}_3$), 1.78 (3H, d, C$\underline{H}_3$CH), m/e (relative intensity %) 361 (6), 207 (12), 154 (78), 124 (33), 84 (100).

b) <u>Sodium (5R,6S)-3-(E-2-acetamidoethenylthio)-6-[(R)-1-(4,5-dimethoxycarbonyltriazol-1-yl)ethyl]-7-oxo-1-azabicyclo[3.2.0.] hept-2-ene-2-carboxylate</u>

(e11) →

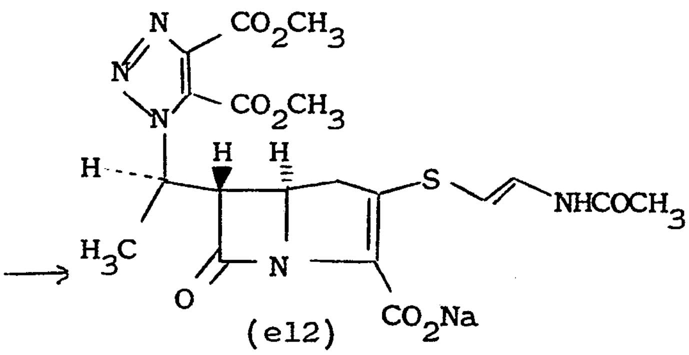

(e12)

The ester (e11) (5 mg) was hydrogenolysed by the procedure described in Example 3b to yield an aqueous solution containing the title compound (e12) (2 mg), $\lambda_{max}$ ($H_2O$) 307 nm, 224 nm.

Example 6

a) p-Nitrobenzyl (5R,6S)-3-(E-2-acetamidoethenyl thio)-6-[(R)-1-(4-p-nitrobenzyloxycarbonyltriazol-1-yl)ethyl]-7-oxo-1-azabicyclo [3.2.0.] hept-2-ene-2-carboxylate

(e10)

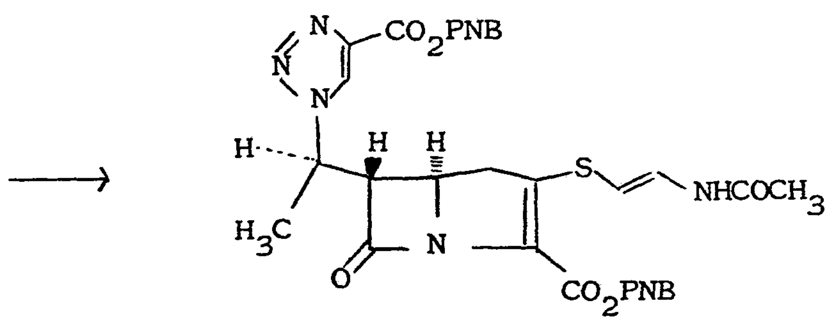

(e13)

The azido derivative (e10) (60 mg; 0.127 mM) was suspended in dry toluene (10 ml) and refluxed for 3 hours with p-nitrobenzyl propiolate (52 mg; 0.253 mM). The solution was then allowed to cool and the precipitate collected by filtration, washing well with toluene. The resulting solid (38 mg) was digested in chloroform/acetonitrile and re-filtered, washing with dry diethyl ether. The title compound (e13) (19 mg) was obtained as a white solid, $\nu_{max}$ (KBr) 3400, 1780, 1730, 1698, 1620, 1610 $cm^{-1}$, $\lambda_{max}$ ($CH_3CN$) 323 nm (Em 16,970) 264 nm (Em 26,340) 215 nm (Em 32,890), $\delta_H$ ($d_7$-DMF) 10.48 (1H, d, N<u>H</u>); 9.12 (1H, S, triazole C<u>H</u>), 8.32 (4H, 2 x d, aromatic protons); 7.83 (4H, d, aromatic protons), 7.20 (1H, dd, C=C<u>H</u>-N), 5.98 (1H, d, C=C<u>H</u>-S), 5.3-5.7 (5H, q + s + m, aromatic protons + 8-C<u>H</u>), 4.25 (2H, dd + m, $J_{56}$ 3.0Hz, $J_{68}$ 8.9 Hz, 6-C<u>H</u> + 5-C<u>H</u>), 3.28 (2H, <u>AB</u>X, 4-C$H_2$), 2.02 (3H, S, CO$CH_3$), 1.81 (3H, d, $CH_3$CH).

b) <u>Disodium (5R,6S)-3-[(E)-2-acetamidoethenylthio]-6-[(R)-1-(4-carboxytriazol-1-yl)ethyl]-7-oxo-1-azabicyclo [3.2.0.] hept-2-ene-2-carboxylate</u>

(e13) 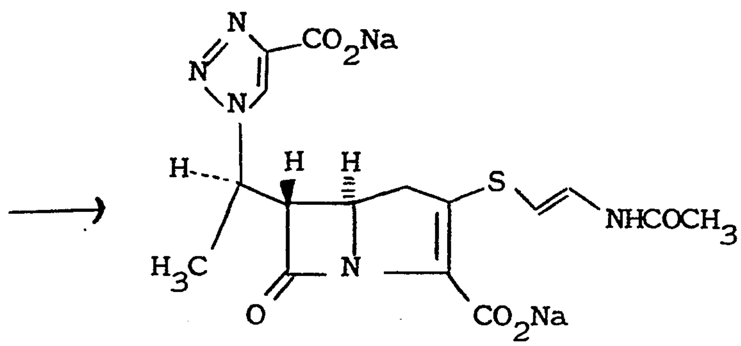

(e15)

The ester (e13) (6 mg) was hydrogenolysed by the procedure described in Example 1b to yield an aqueous solution containing the title compound (e15). This solution was estimated to contain 3.0 mg product based on Em 14,000 at $\lambda_{max}$ 309 nm in the u.v. spectrum.

Example 7

a) p-Nitrobenzyl (5R,6S)-3-(E-2-acetamidoethenylthio)-6-[(R)-1-[4-(2-[p-nitrobenzyloxycarbonylamino]ethyloxycarbonyl)triazol-1-yl]ethyl]-7-oxo-1-azabicyclo [3.2.0.] hept-2-ene-2-carboxylate

(e10) 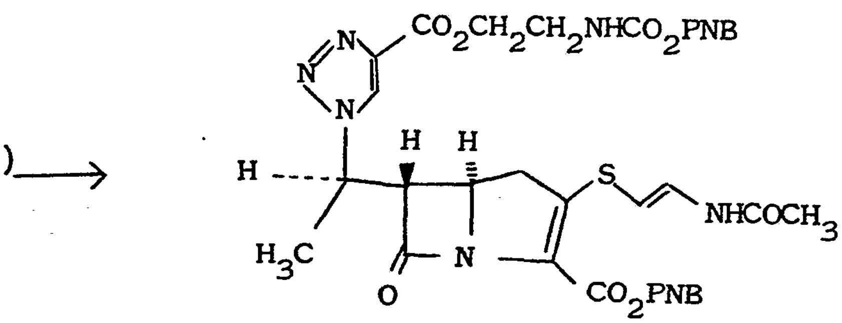

(e14)

The azido derivative (e10) (60 mg; 0.127 mM) was suspended in dry toluene (50 ml) and refluxed for 24 hours with 2-(N-p-nitrobenzyloxycarbonyl)aminoethyl propiolate (54 mg; 0.185 mM). The solvent was then removed at reduced pressure and the resulting pale yellow solid was purified by column chromatography over silica gel (4 gm). Elution with 5% ethanol/ethyl acetate gave the title compound (e14) as a white solid. This solid was digested in dry diethyl ether and collected by filtration (17 mg), $\nu_{max}$ ($CHBr_3$) 3300-3400 (broad), 1775, 1680-1720 (broad), 1618, 1602, 1518, 1342 $cm^{-1}$, $\lambda_{max}$ ($CH_3CN$) 322 nm (Em 15,605), 265 nm (Em, 26,275), 215 nm (Em 33,890), $\delta_H$ ($d_7$-DMF) 10.47 (1H, d, N<u>H</u>), 8.96 (1H, s, triazole -C<u>H</u>), 8.30 (4H, 2 x d, aromatic protons), 7.83 (2H, d, aromatic protons), 7.68 (3H, d + broad res., aromatic protons + N<u>H</u>), 7.20 (1H, dd, C=C<u>H</u>-NH), 5.97 (1H, d,

$C{=}C\underline{H}{-}S$), 5.32-5.62 (3H, q + m, $C\underline{H}_2Ar$ + 8-$C\underline{H}$), 5.28 (2H, S, $C\underline{H}_2Ar$), 4.42 (2H, t, $CO_2C\underline{H}_2$), 4.18-4.38 (2H, dt + dd, $\underline{J}_{56}$2.5Hz, $\underline{J}_{68}$ 8.0Hz, 5-$C\underline{H}$ + 6-$C\underline{H}$), 3.56 (q, $CH_2C\underline{H}_2NH$), 3.28 (2H, $\underline{AB}X$, 4-$C\underline{H}_2$, $\underline{J}_{4a5}$ 9.75 Hz, $\underline{J}_{4b5}$ 8.5 Hz, $\underline{J}_{4a4b}$ 18.25 Hz), 2.03 (3H, s, $COC\underline{H}_3$), 1.79 (3H, d, $C\underline{H}_3CH$).

b) (5R,6S)-3-[(E)-2-acetamidoethenylthio]-6-[(R)-1-[4-(2-aminoethyloxycarbonyl)triazol-1-yl]ethyl]-7-oxo-1-azabicyclo [3.2.0.] hept-2-ene-2-carboxylic acid

(e14)

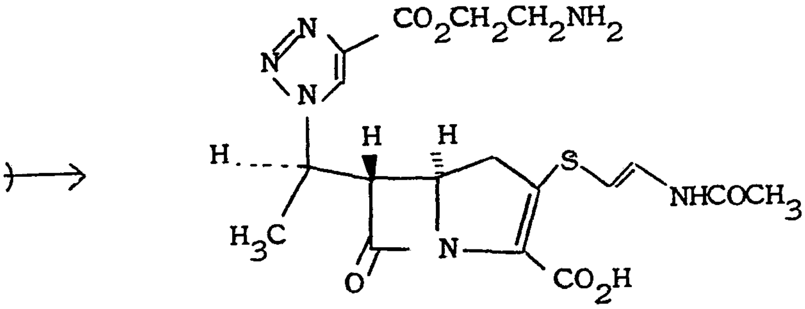

(e16)

A solution of the ester (e14) (6 mg) in 1,4-dioxan (5 ml), water (0.75 ml) and 0.05 M phosphate buffer (1 ml) was hydrogenated in the presence of 5% palladium on carbon catalyst (12 mg) for 3 hours. The suspension was then filtered over Celite, washing with water (15 ml). The filtrate was concentrated to approximately 15 ml and washed with ethyl acetate (3 x 25 ml). The resulting aqueous solution was estimated to contain 1.9 mg of the title compound (e16) based on Em 14,000 at $\lambda_{max}$ 308 nm in the u.v. spectrum.

Example 8

(5R,6S)-3-(E-2-Acetamidoethenylthio)-6-[(R)-1-aminoethyl]-7-oxo-1-azabicyclo[3.2.0]hept-2-ene-2-carboxylic acid

(e3)

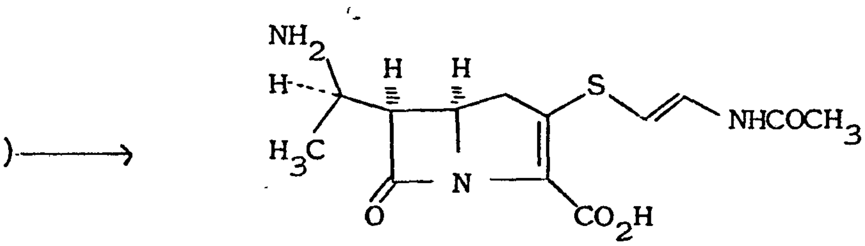

(e17)

The azido - derivative (e3) (4 mg) was dissolved in 1,4-dioxan (4 ml), water (0.6 ml) and pH 7.0, 0.05 M phosphate buffer (0.75 ml) and shaken with hydrogen for 2 hours in the presence of 5% palladium on carbon (8 mg). The suspension was filtered over Celite, washing well with water (25 ml). The filtrate was concentrated to approximately 20 ml and washed with ethyl acetate (3 x 50 ml). The aqueous solution was further concentrated to about 5 ml and chromatographed over Biogel P2, eluting with water. Fractions containing the title compound (e17) were identified by the chromophore at $\lambda_{max}$ ($H_2O$) 300 nm in the uv spectrum and were combined to yield an aqueous solution of the product (0.67 mg), $\lambda_{max}$ ($H_2O$) 300 nm, 226 nm.

BIOLOGICAL DATA

| ORGANISM | COMPOUND OF EXAMPLE NO. 4 MIC (μg/ml) Broth[1] | 6 MIC (μg/ml) Broth[1] | 7 MIC (μg/ml) Broth[1] |
|---|---|---|---|
| Citrobacter freundii E8 | 1.6 | 25 | 0.8 |
| Enterobacter cloacae N1 [T753] | 3.1 | 25 | 1.6 |
| Escherichia coli 0111 [E8] | 3.1 | 12.5 | 1.6 |
| Escherichia coli JT39 [E96] | 3.1 | 3.1 | 0.8 |
| Escherichia coli ESS | 1.6 | 1.6 | 0.8 |
| Klebsiella aerogenes A [T767] | 3.1 | 1.6 | 3.1 |
| Proteus mirabilis 977 [13] | 12.5 | 12.5 | 25 |
| Proteus morganii 1580 | 25 | 12.5 | - |
| Proteus rettgeri WM16 | 6.2 | 3.1 | 6.2 |
| Proteus vulgaris W091 | 6.2 | 12.5 | 25 |
| Pseudomonas aeruginosa A [11] | 100 | 50 | 100 |
| Salmonella typhimurium CT10 | 6.2 | 1.6 | 1.6 |
| Serratia marcescens US20 | 6.2 | 6.2 | 3.1 |
| Shigella sonnei MB11967 | 12.5 | 3.1 | 1.6 |
| Bacillus subtilis A | 3.1 | 6.2 | 0.8 |
| Staphylococcus aureus Oxford | 6.2 | 6.2 | 0.4 |
| Staphylococcus aureus Russell [MB9] | 6.2 | 6.2 | 0.4 |
| Staphylococcus aureus 1517 | >100 | 25 | 12.5 |
| Streptococcus faecalia I | 100 | 100 | 50 |
| Streptococcus pneumoniae CN33 | 1.6 | NG | NG |
| Streptococcus pyogenes CN10 | 1.6 | NG | 0.4 |

Claims

1. A compound of the formula (I) or a pharmaceutically acceptable salt or in vivo hydrolysable ester thereof:

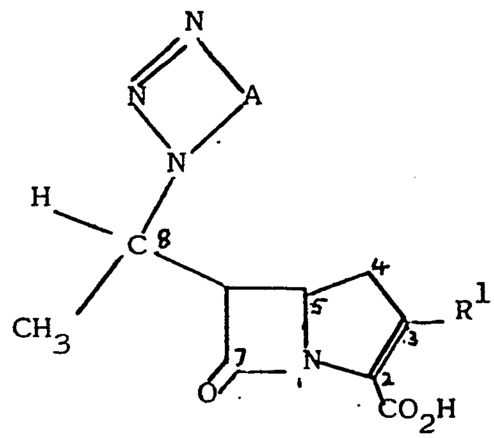

(I)

wherein $R^1$ is hydrogen, an organic radical or group $S(O)_nR^2$ wherein $R^2$ an organic radical and n is zero or one; and A is a divalent two atom radical forming the residue of a five membered ring wherein one of the atoms is optionally substituted carbon and the second atom is an optionally substituted carbon or nitrogen atom.

2. A compound as claimed in claim 1 wherein the divalent two atom radical A completes an optionally mono- or disubstituted triazole or dihydrotriazole ring.

3. A compound as claimed in claim 1 or claim 2 wherein the divalent two atom radical A completes a mono- or di-substituted triazole ring.

4. A compound as claimed in any one of claims 1 to 3 wherein $R^1$ is acetamidoethylthio or acetamidoethenylthio.

5. A compound as claimed in any one of claims 1 to 4 wherein the hydrogen atoms at the C-5 and C-6 carbon atoms are in the trans configuration with the configuration 5R, 6S.

6. A compound as claimed in any one of claims 1 to 4 wherein the hydrogen atoms at the C-5 and C-6 carbon atoms are in the cis configuration with the configuration 5R, 6R.

7. A compound as claimed in any one of claims 1 to 6 having formula (II) or a pharmaceutically acceptable salt or <u>in vivo</u> hydrolysable ester thereof:

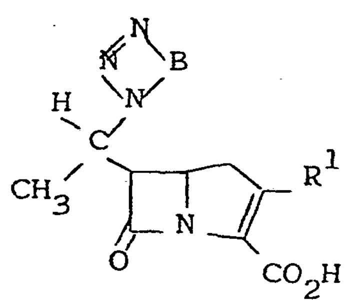

(II)

wherein $R^1$ is as defined in claim 1 and B is a divalent radical of structure:

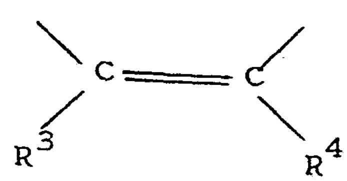

wherein $R^3$ represents a carboxylic acid group or an ester or amide derivative thereof or a nitrile group; and $R^4$ represents hydrogen, an optionally substituted hydrocarbon or heterocyclic group, a carboxylic acid group or an ester or amide derivative thereof or a nitrile group.

8. A compound as claimed in claim 7 wherein $R^3$ is a carboxylic acid group, or a salt, or methyl, ethyl, p-nitrobenzyl, 2-amino-ethyl or 2-(p-nitrobenzyloxycarbonylamino)ethyl ester thereof.

9. A compound as claimed in claim 7 or claim 8 wherein $R^4$ is hydrogen.

10. A pharmaceutical composition which comprises a compound as claimed in claim 1 and a pharmaceutically acceptable carrier.

11. A process for the preparation of a compound as claimed in claim 1 which process comprises reacting a compound of formula (III):

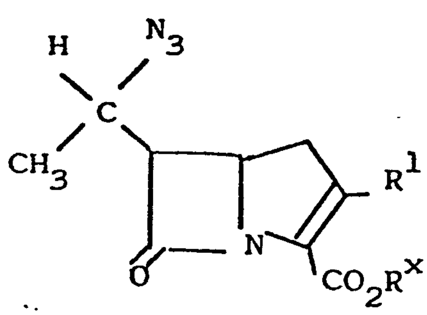

(III)

wherein $R^1$ is as defined with respect to formula (I) and wherein any reactive groups may be protected and $R^x$ is a carboxy protecting group.

with a dipolarophile; and thereafter where necessary carrying out one or more of the following steps:

i) removing any protecting groups on $R^1$, or the dipolarophile,

ii) removing the carboxy protecting group $R^x$,

iii) converting a free acid to an *in vivo* hydrolysable ester or a phrmaceutically acceptable salt.

12. A compound of formula (VIII) or a pharmaceutically acceptable salt or *in vivo* hydrolysable ester thereof:

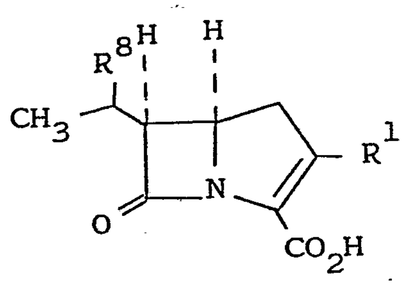

(VIII)

wherein $R^1$ is as defined with respect to formula (I) hereinbefore and $R^8$ is an azido or amino group.